# EUROPEAN PATENT SPECIFICATION

(11) **EP 1 919 357 B1**
(45) Date of publication and mention of the grant of the patent: **27.03.2013**
(21) Application number: 06795881.9
(22) Date of filing: 01.09.2006
(51) Int. Cl.: A61B 5/053, A61B 5/103, G06Q 30/00

(54) **METHODS FOR RETAIL MEASUREMENT OF SKIN MOISTURE CONTENT**
VERFAHREN ZUR MESSUNG DES FEUCHTIGKEITSGEHALTS DER HAUT FÜR DEN HANDEL
PROCEDES DE MESURE DU TAUX D'HYDRATATION DE LA PEAU DANS LES MAGASINS

(30) Priority: 02.09.2005 US 713966 P
(43) Date of publication of application: 14.05.2008
(73) Proprietor: The Procter & Gamble Company, Cincinnati, OH 45202 (US)
(72) Inventor: SHERMAN, Faiz, Feisal, Mason, Ohio 45040 (US); GARTSTEIN, Vladimir, Mason, Ohio 45040 (US); MOORE, David, Burton, Hamilton, Ohio 45013 (US); MARGRAF, Carl, Hinz, III, 60325 Frankfurt Am Main (DE); FISHER, Brian, Keith, Cincinnati, Ohio 45249 (US); STAMPER, Adam, Michael, CH-1205 Geneva (CH); KELLY, Colin, Lowfell Gateshead NE9 6DT (GB); ALEXANDER, Claire, London SW6 2PP (GB)
(74) Representative: Boubel, Thomas
(86) International application number: PCT/IB2006/053078
(87) International publication number: WO 2007/026337

(56) References cited:
- WO-A-98/30189
- WO-A-2004/042510
- US-A1- 2003 078 971

## Description

### FIELD OF THE INVENTION

The present invention relates generally to methods for providing a personal care regimen to a consumer. The present invention also relates to methods for measuring skin moisture content of a consumer. Another aspect of the present invention, relates to methods for measuring scalp moisture content of a consumer in a retail location.

### BACKGROUND OF THE INVENTION

Skin is subject to insults by many extrinsic and intrinsic factors. Extrinsic factors include ultraviolet radiation (e.g., from sun exposure), environmental pollution, wind, heat or infrared radiation (IR), low humidity, harsh surfactants, abrasives, and the like. Intrinsic factors include chronological aging and other biochemical changes from within the skin. Whether extrinsic or intrinsic, these factors result in visible signs of skin aging and environmental damage, such as wrinkling and other forms of roughness (including increased pore size, flaking and skin lines), and other histological changes associated with skin aging or damage. Additionally, the water content of the stratum corneum has a profound influence on the appearance, flexibility, texture, and dryness of the skin, and also on the absorption of drugs and other molecules into and through the skin. The stratum corneum is the outermost layer of the epidermis, and comprises the surface of the skin

Methods of treating the skin generally involve the application of at least one of a variety of appropriate treatments. Such treatments may be selected to provide or to restore certain desired physical or cosmetic characteristics to the skin or scalp. However, unless an appropriate treatment is selected, the desired physical or cosmetic characteristic may not be obtained.

In the case of treating skin, such as the scalp, treatments generally include shampoos, conditioners, colorants, styling compositions, and the like. Manufacturers of these scalp treatments may provide multiple versions of a type or brand of scalp treatment, wherein each of the multiple versions is specifically designed to target a need or demand which is characteristic of a specific consumer segment and which may be based on physical or cosmetic differences of the scalp generally found between respective consumer segments. For example, a single brand shampoo may offer a first version designed to treat flakes and a second version designed to treat dryness, both conditions associated with dandruff.

However, when a consumer is faced with the task of selecting a scalp treatment from among the multiple versions of a scalp care brand, the consumer may unknowingly select a version which is not designed to provide the characteristics desired by the consumer. In such a case, the consumer may be dissatisfied with the results of the selected version of the scalp care brand. As a result of the consumer's dissatisfaction, the consumer subsequently may refuse to select any of the versions of that same scalp care brand even though another version of that scalp care brand may provide the consumer's desired scalp and/or hair characteristics. The occurrence of such circumstances, in turn, may lead to unnecessary loss of sales of the particular scalp care brand for the manufacturer.

Furthermore, retail shopping environments tend to be impersonal. The consumer is left basically alone to pick and choose the appropriate skin or hair care treatments that are most suited to his or her own needs and preferences. Even when a salesperson is available to assist the consumer with or make recommendations for treatment selections, the salesperson's assistance and recommendations are based upon a limited and/or subjective knowledge of the consumer and the consumer's treatment needs.

Methods of measuring moisture content have been developed in the past to determine the moisture level of skin, and have relied on various techniques including resistance and capacitance measurements to obtain the desired indication. WO 2004/042510 A relates to a method of providing an individualized hair care program to a customer in a retail shopping environment. More specifically, WO 2004/042510 A is directed to a method of providing an individualized hair care program to a customer in a retail shopping environment, which entails obtaining personal information from the customer in the retail shopping environment, using the information to create an individualized hair care program for the customer, and providing the individualized hair care program to the customer in the retail shopping environment. However, none of these devices are made integral in the purchasing experience by combining a moisture measuring system with a product line of hair care products.

### SUMMARY OF THE INVENTION

In one embodiment, the present invention is directed to a method for measuring scalp moisture content of a consumer. The method comprises the steps of:
a) collecting input information from said consumer regarding a plurality of characteristics associated with scalp condition of said consumer;
b) providing a moisture meter for measuring said scalp moisture content;
c) using said moisture meter to obtain at least one measured moisture content value from a position on scalp of said consumer;
d) obtaining a second measured moisture content value at a position off said scalp;
   characterized in that the method further comprises the steps of:
e) comparing said first measured moisture content value with said second measured moisture content value to obtain a measured moisture content differential;
f) correlating said measured moisture content differential to a scalp moisture value for said scalp;
g) using said input information and said scalp moisture value to identify at least one hair care product for said consumer; and
h) providing said consumer with information regarding the utilization of said at least one hair care product.

These and other features, aspects and advantages of the present invention will become evident to those skilled in the art from a reading of the present disclosure.

### BRIEF DESCRIPTION OF THE DRAWINGS

While the specification concludes with claims which particularly point out and distinctly claim the invention, it is believed the present invention will be better understood from the following description taken in conjunction with the accompanying drawing.
FIG. 1 is a flow diagram of the process of measuring scalp moisture content of a consumer in accordance with one embodiment of the present invention;
FIGS. 2 and 3 are screen shots of screens which may be used and are examples of block 120 within FIG. 1;
FIGS. 4 and 5 are screen shots of screens which may be used and are examples of block 130 within FIG. 1;
FIGS. 6 and 7 are screen shots of screens which may be used and are examples of block 140 within FIG. 1;
FIG. 8 is a screen shot of a screen which may be used and is an example of block 150 within FIG. 1;
FIGS. 9 and 10 are screen shots of screens which may be used and are examples of block 160 within FIG. 1; and
FIG. 11 is a screen shot of a screen which may be used and is an example of block 170 within FIG. 1.

### DETAILED DESCRIPTION OF THE INVENTION

As used herein, the term "skin" refers to the membranous tissue forming the external covering of a mammalian body including, for example, the external covering of the face, neck, chest, back, arms, hands, legs and scalp.

As used herein, "moisture content" refers to the percentage of water present in the skin.

As used herein, "control area" refers to an area of the skin that is exposed to the environment and convenient for measurement.

As used herein, "test area" refers to the area of skin that a consumer wishes to have tested in order to select an appropriate skin treatment for use by consumer on the area.

According to the present invention, methods are described for measuring the moisture content of scalp. The determination of moisture content in scalp, is used to quantify various physical and cosmetic characteristics of the skin. For example, a scalp with a low moisture content is unhealthy and may exhibit signs of dandruff, including flakes, dryness, tightness, itchiness and/or redness/irritation. Skin hydration is a function of its normal biological activity that results in continuous moisture flux from within the body to the environment. The improvement of skin barrier function results in greater skin hydration and less moisture loss. As a result, the physical and cosmetic characteristics of the skin may be improved, for example, with treatments that restore skin to normal conditions and improve its barrier function. Improvement of barrier function, in turn, also results in protecting the skin from environmental, physical, chemical, or biological insults and results in an overall improvement in skin health.

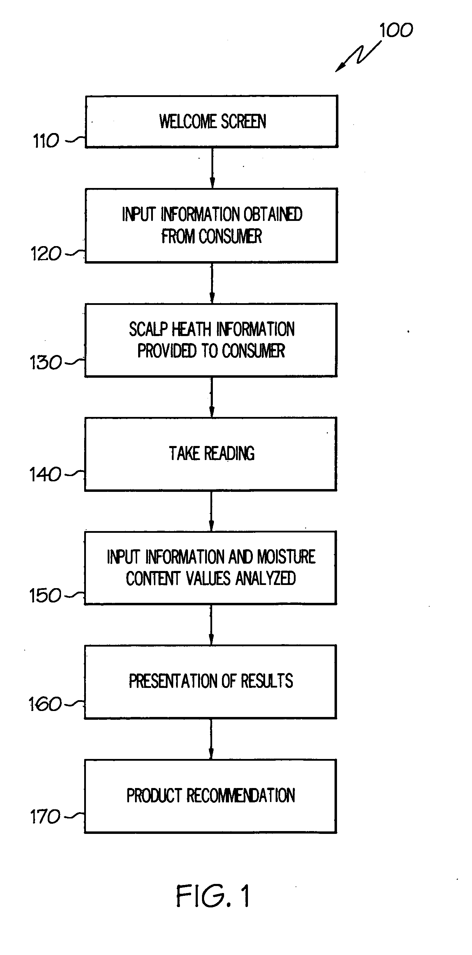

Referring now to FIG. 1, method 100 for measuring scalp moisture content of a consumer comprises the steps of: step 110, welcoming screen; step 120, obtaining input information from consumer; step 130, providing scalp health information to consumer; step 140, taking reading; step 150, analyzing input information and moisture content values; step 160, presenting results to consumer; and step 170, recommending product to consumer. Each of steps 110 to 170 may be represented by screen shots that are intended to give and receive information to a consumer for purposes of executing method 100. These screen shots may be displayed on an imaging device, which may include, but is not limited to, a computer screen, television monitor, light radiating display (for example, PDA), and print materials.

In one embodiment, scalp moisture content measuring method 100 is carried out in a retail location with the aid of a computer system capable of performing automated data analysis for the purpose of analyzing and recommending hair care products, personal care products, and beauty care products, in the form of, lotions, creams, gels, tonics, after shave, sticks, sprays, ointments, pastes, powders, mousse, shampoos, conditioners, oils, colorants, and biomedical and dermatological treatments.

Nonlimiting examples of biomedical and dermatological treatments include prescription skin care treatments, laser treatment, chemical peel, dermabrasion, electrical stimulation, botox treatments, and exfoliating pads and cloths.

In one example, the automated data analysis may be performed using software. Off-the-shelf software may be used. In another example, the software may be modified to meet the specific needs of the particular system and/or user. As used herein, "retail location" includes business establishments stocked with items for sale or with standard services for sale, where consumers go to examine goods and services with the possible intent to buy for their personal or household use. Examples of such retail shopping environments include, without limitation, department stores, shopping malls, shopping centers, open air markets, kiosks, drug stores, mass merchandisers, specialty shops, grocery stores, hair care salons, and convenience stores. Typically, consumers may come and go freely during the normal operating business hours of these retail shopping environments. However, persons skilled in the art would readily appreciate that the method may be used in a variety of other locations without departing from the scope of the present invention. For example, the method could be used in salon, barber shops, doctors' offices, dermatologists' offices, at home, clinical research centers, hospitals, laboratories and public areas.

In one embodiment of the present invention, step 110 contains a welcome screen which identifies the system and the associated hair care products, for example, HEAD & SHOULDERS® products.

Still referring to FIG. 1, step 120 depicts the step of collecting input information from a consumer. In one embodiment, input information may be collected from a consumer by questioning means, that is, by the consumer's answering questions, which are asked of him or her, either orally or in written form, or electronically, such as via a computer terminal or other imaging device. The questioning means may be an interviewer asking oral questions of the consumer, for example, an in-store interview, a written questionnaire on which the consumer writes answers to the written questions, or an electronic questionnaire viewed by the consumer on a computer screen or other imaging device and for which the consumer submits answers to the questions by typing on a keyboard, touching a responsive screen, speaking an answer, or the like. In another embodiment, the information may be collected from a consumer through the use of an interactive site via the internet. The consumer's response to the questions may then be used to assist in recommending an appropriate hair care product for the consumer. These questions may be focused on a plurality of hair and scalp characteristics and/or preferences, including, for example, hair care habits, hair care product use preferences, skin and scalp sensitivity, skin and scalp problems and concerns, and hair care questions.

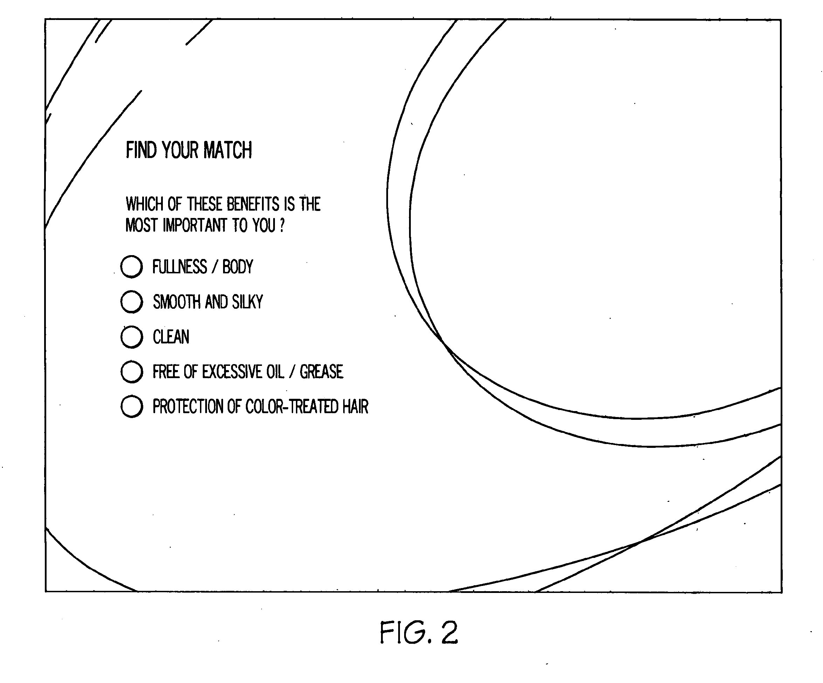

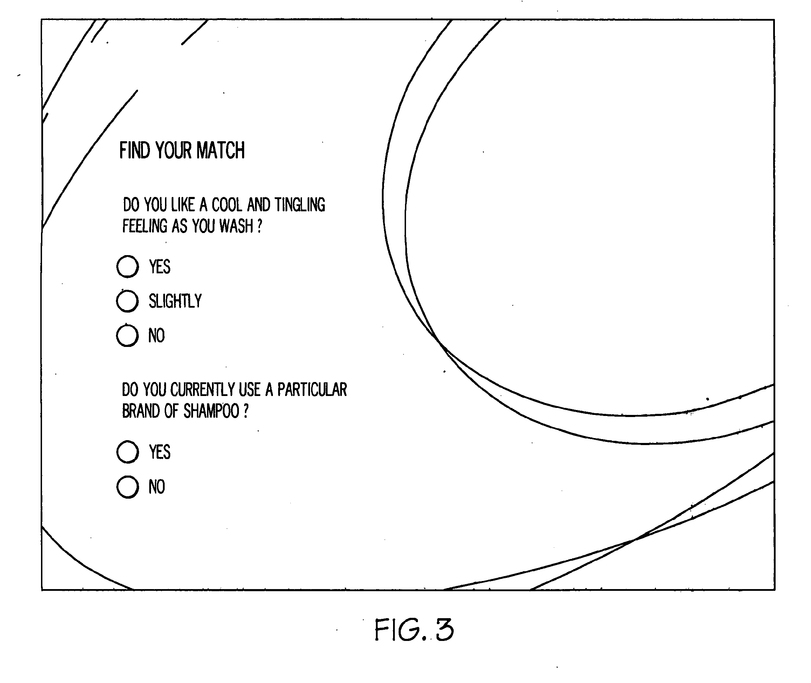

Referring to FIGS. 2 and 3, scalp moisture content measuring method 100 contains step 120. Generally illustrated is text in the form of questions to the consumer such as: Which of these benefits is the most important to you?; Do you like a cool and tingling feeling as you wash?; Do you currently use a particular brand of hair care product? The intent of step 120 is for the purpose of assisting in the making of proper hair care recommendations. The precise manner and wording chosen to collect this information from the consumer may vary depending on local custom, the comfort level of consumers in discussing personal care characteristics, for example, skin, hair and scalp, and the meaning associated with terms which may be used in different parts of the world to collect information desired. It is to be further understood that the methods of the present invention are not to be limited to any one type of question asking methodology or philosophy.

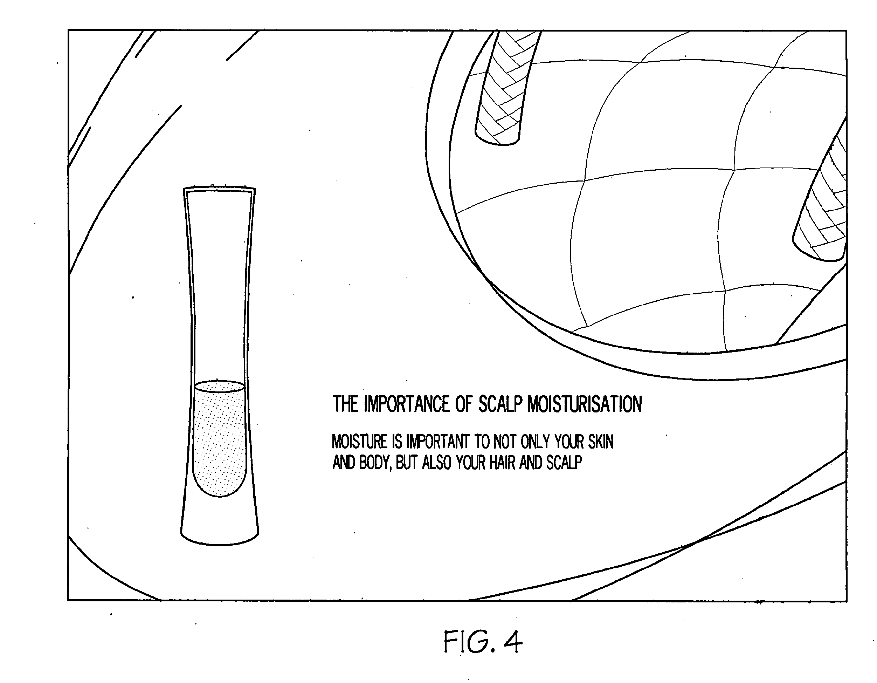

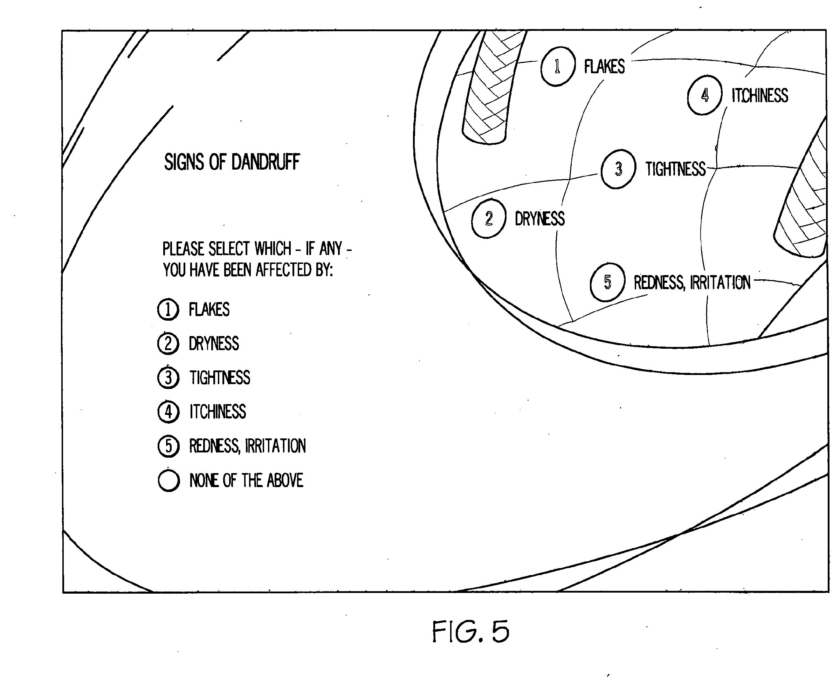

Referring to FIGS. 4 and 5, scalp moisture content measuring method 100 may further contain step 130, that is, providing a consumer with educational information regarding scalp health, for example, scalp moisturization and signs of dandruff Referring now to FIG. 4 wherein the consumer views content regarding the importance of scalp moisturization. Generally illustrated in FIG. 4 is a close-up or magnified depiction of a segment of human scalp, including hair follicles extending therefrom. Text may be included in this illustration as a warning or caution to alert the user of the need to assure proper moisturization of the scalp. Referring now to FIG. 5 wherein the consumer views content regarding the signs of dandruff. Generally illustrated in FIG. 5 is a close-up or magnified depiction of a segment of human scalp, including hair follicles extending therefrom. Text may be included in this illustration, for example, asking the consumer if they have been affected by certain dandruff conditions, such as, "flakes, dryness, tightness, itchiness, redness, irritation, none of the above." One skilled in the art will readily appreciate that any words which represent dandruff conditions may be used in this invention.

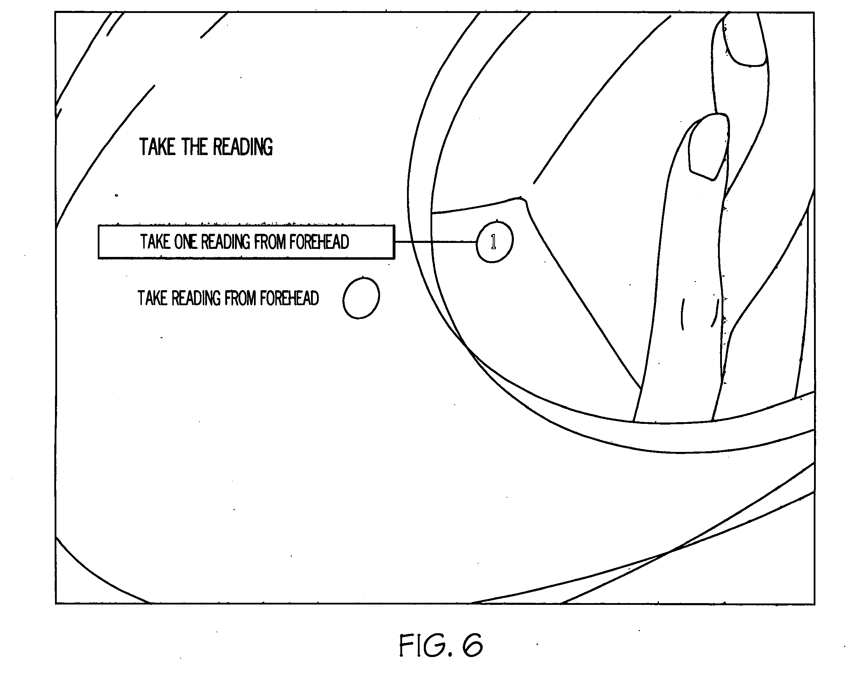

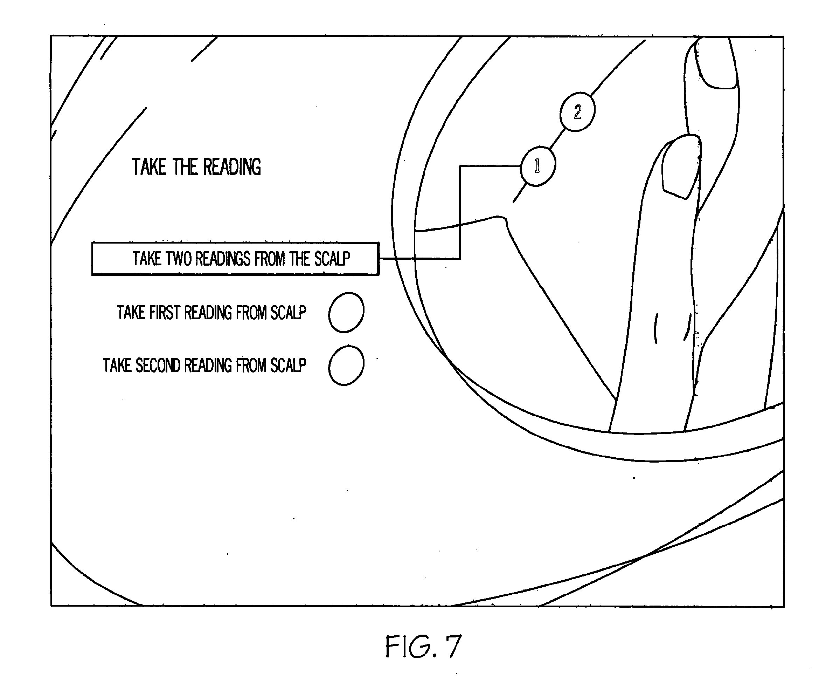

Referring to FIGS. 6 and 7, scalp moisture content measuring method 100 contains step 140, that is, taking/obtaining at least one moisture content value from a position on the scalp of a consumer using a moisture meter for measuring scalp moisture content. Generally illustrated in FIGS. 6 and 7 is a close-up or magnified depiction of the head of a consumer, including the forehead and scalp portions. Text may be included in these illustrations as a prompt to instruct the consumer / in-store consultant to take measurements. Step 140 may be performed by the consumer themselves, or it may be performed on the consumer by another, for example, a beauty counselor or hair care consultant. In one embodiment, the consumer is prompted to take/obtain two measured moisture content values, for example, a first measured moisture content value at a position off the scalp of a consumer and a second measured moisture content value at a position on the scalp. In one example, the off the scalp location may includes, for example, forehead skin, cheek, chin, ear and arm. With respect to the at least second measured moisture value, several such measurements at various positions on the scalp can be produced. In one example, the moisture meter for measuring scalp moisture content is used to obtain one or more additional measured moisture content values at various locations on the scalp. As each measured moisture content value is captured, the values are displayed in a suitable manner, for example, on an LED display on the moisture meter. In one example, the measured moisture content values are stored in a programmable integrated circuit.

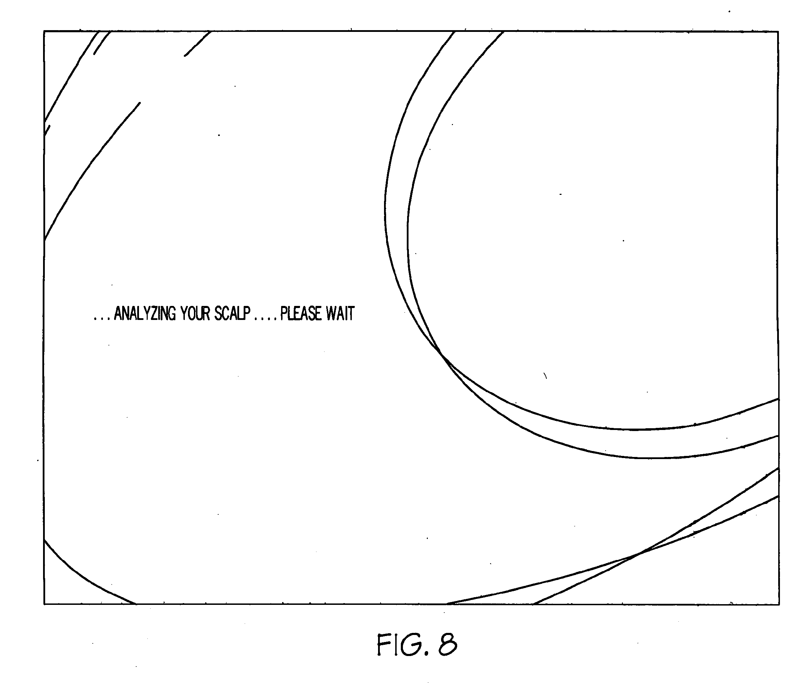

After having collected the input information and measured moisture content values from a consumer as described in steps 120 to 140 , scalp moisture content measuring method 100 analyzes this information and corresponding values as depicted in step 150. Referring to FIG. 8, the collected input information and measured moisture content values are analyzed, for example, the first measured moisture content value is compared with the second measured moisture content value to obtain a measured moisture content differential. Generally illustrated is text advising the consumer that the analysis is being performed. In one example, the scalp moisture content value for the position off the scalp (m1) is compared to the scalp moisture content value for the position on the scalp (m2), to obtain a measured moisture content differential (m2 - m1) or (m1 - m2). In one embodiment, the moisture meter is electrically coupled through a cable to a processing system, such as a conventional PC or laptop computer. In another embodiment, the moisture meter is electrically coupled to a programmable integrated circuit. In yet another embodiment, the moisture meter is electrically coupled to a computer wirelessly. The processing system is operable to convert the measured moisture content values generated by the meter into a measured moisture content differential. In another embodiment, the measured moisture content differential may be determined manually using the formula (m2 - m1) or (m1 - m2).

If additional scalp moisture values are obtained at positions on the scalp, all of these values are compared to one another and the measurement with the largest value, or lowest value or mean value is then compared with the moisture content value for the position off the scalp. The result of this comparison is a measured moisture content differential profile. In one embodiment, a measurement differential from about 0 MHz to about 0.05 MHz, represents a healthy scalp condition (high moisture level); a measurement differential from about 0.05 MHz to about 0.5 MHz represents a moderate scalp condition (medium moisture level); and a measurement differential greater than about 0.5 MHz represents an unhealthy scalp condition (low moisture level).

Still referring to FIG. 8, the moisture content differential is correlated to a scalp moisture value. In one embodiment, the processing system described above is operable to convert the frequency differential into a scalp moisture value. as shown in table I.

**TABLE I**

| Measured Signal Difference between m 1 and m2 (MHz) | Scalp Moisture Value | Scalp Condition |
|---|---|---|
| >=0.9 | 5 | D |
| 0.79-0.89 | 10 | D |
| 0.6 - 0.78 | 20 | D |
| 0.54 - 0.67 | 30 | D |
| 0.43 - 0.53 | 40 | D |
| 0.34 - 0.42 | 50 | M |
| 0.25 - 0.33 | 60 | M |
| 0.15 - 0.24 | 70 | M |
| 0.06 - 0.14 | 80 | M |
| 0.04 - 0.05 | 90 | H |
| <= 0.03 | 95 | H |

In one embodiment, the scalp moisture value is from about 0 to about 99, and in another embodiment, from about 5 to about 95. Such a value is based upon the relative differences between the measured values for the off the scalp position and the on the scalp position. As such, the scalp serves as its own control. The scalp moisture value may also be represented by such terms as "healthy (H)", "moderate (M)", and "dry (D)," however, any words or a numbered grade scale which depict increasing or decreasing quantities of scalp moisture levels may be used in the invention.

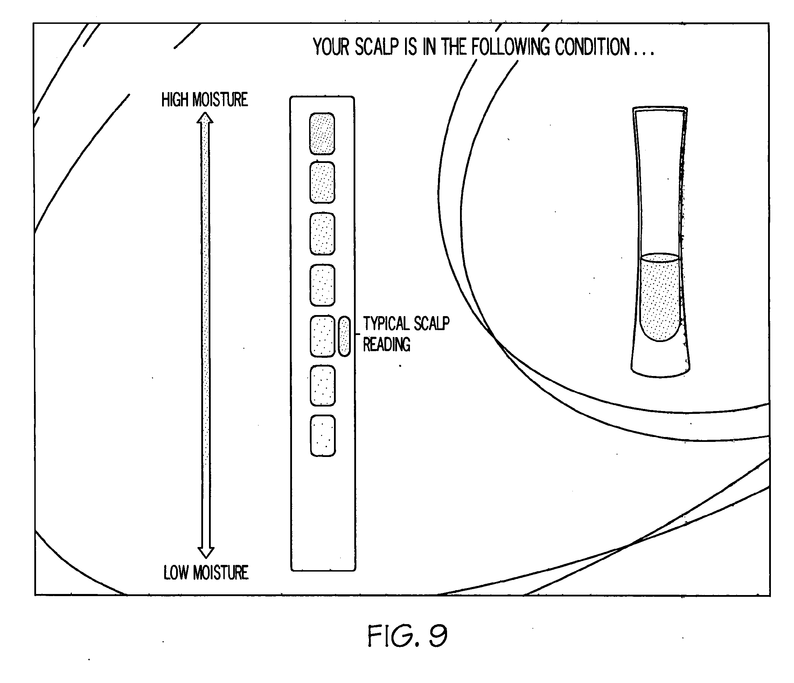

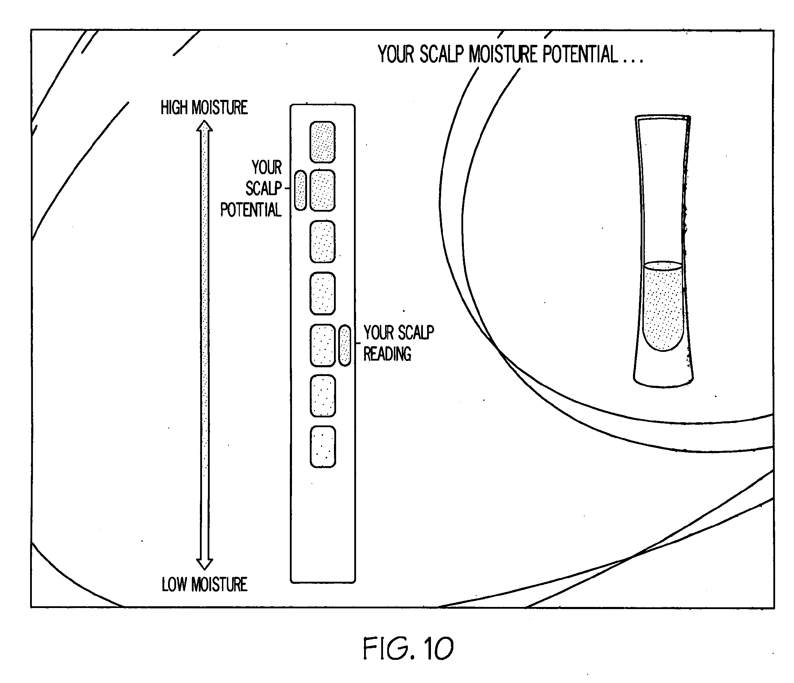

Referring to FIGS. 9 and 10, scalp moisture content measuring method 100 may further contain step 160, that is, giving the results of the scalp moisture content measurements to the consumer. Referring now to FIG. 9 wherein the consumer views content regarding the moisture content of a "typical scalp". Generally illustrated in FIG. 9 is colored water being poured into a measuring jar up to a certain level that corresponds to a scalp moisture level. Text may be included in this illustration to depict a range of moisture levels from low to high. Referring to FIG. 10. wherein the consumer views content regarding their own scalp moisture content level and their scalp moisture potential. Generally illustrated in FIG. 10 is colored water being poured into a measuring jar up to a certain level that corresponds to the consumer's scalp moisture level. Text may be included in this illustration to depict a range of moisture values from low to high.

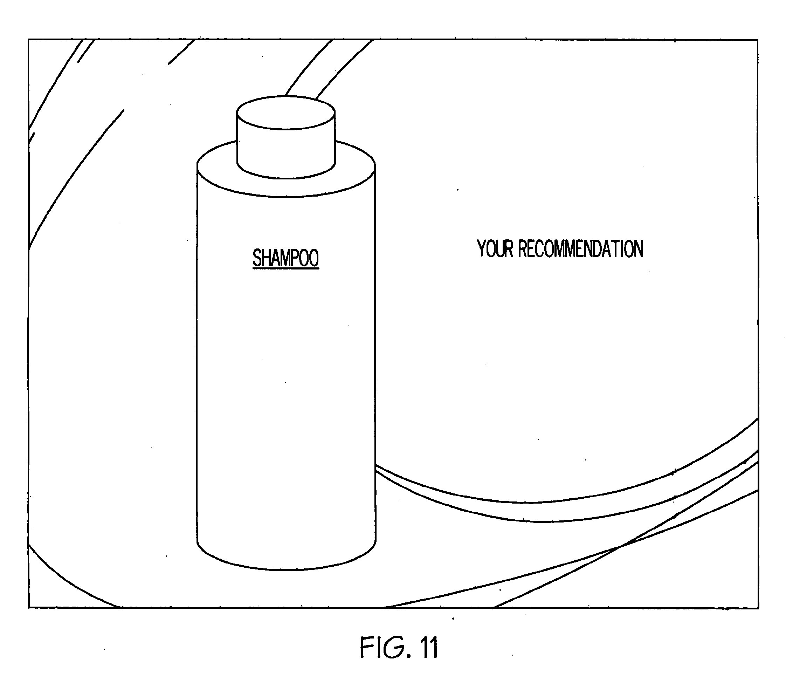

After having analyzed the input information and measured moisture content values from a consumer as described above, step 170 of scalp moisture content measuring method 100 calls for recommending at least one hair care product, thereby providing feedback information to the consumer identifying hair care products that will be effective in improving hair and scalp health. Referring to FIG. 11, wherein the consumer views content regarding a particular hair care product recommendation to a consumer. Generally illustrated is a depiction of a hair care product, for example, HEAD & SHOULDERS® extra volume shampoo. In one example, the product recommendation may be provided to the consumer in the form of, for example, a written report, print-out, or graphical interface outputted to an imaging device. In another example, the product recommendation may be provided to the consumer through a counseling session with a beauty counselor or hair care consultant. Exemplary hair care products may include, without limitation, shampoos, conditioners, oils, colorants, and styling compositions.

Step 170 of the present invention may further comprise recommending more than one hair care product to a consumer. For example, steps 120 to 150 may indicate that a consumer has a dry scalp and as a result, one hair care product is recommended. In another example, steps 120 to 150 may indicate that a consumer has a healthy scalp. As a result, a hybrid recommendation may be made, for example, a hair care product that helps maintain the consumer's healthy condition is recommended along with a hair care product that will help treat scalp concerns if they arise in the future.

Various embodiments of the present invention may be implemented, for example, by operating a computer system to execute a sequence of machine-readable instructions. These instructions may reside in various types of signal bearing media, such as hard drive, flash card, portable memory chip, and main memory. In this respect, another aspect of the present invention concerns a program product, comprising signal bearing media embodying a program of machine-readable instructions, executable by a digital data processor, such as a central processing unit (CPU), to perform method steps. The machine-readable instructions may comprise any one of a number of programming languages known in the art (e.g., Visual Basic, C, C++, etc.).

The CPU may be, for example, a Pentium, 200 mega hertz plus. However, it should be understood that the present invention is not limited to any one make of processor and the invention may be practiced using some other type of a processor such as a coprocessor or an auxiliary processor. An auxiliary storage adapter may be used to connect mass storage devices (such as a hard disk drive) to a computer system. The program need not necessarily all simultaneously reside on the computer system. Indeed, this latter scenario would likely be the case if computer system were a network computer, and therefore, be dependent upon an on-demand shipping mechanism for access to mechanisms or portions of mechanisms that resided on a server. A display adapter may be used to directly connect a display / imaging device to the computer system. A network adapter may be used to connect the computer system to other computer systems.

It is important to note that while the present invention has been described in the context of a fully functional computer system, those skilled in the art will appreciate that the mechanisms of the present invention are capable of being distributed as a program product in a variety of forms, and that the present invention applies equally regardless of the particular type of signal bearing media used to actually carry out the distribution. Examples of signal bearing media include: recordable type media, such as floppy disks, hard disk drives, and CD ROMs and transmission type media, such as digital and analog communications links and wireless devices.

### EXAMPLES

The following examples are given solely for the purposes of illustration and are not to be construed as limitations of the present invention, as many variations of the invention are possible without departing from the spirit and scope of the invention.

### Example 1. Scalp Moisture Content and Dandruff

To determine a correlation between moisture content of scalp and dandruff, the scalp moisture content of consumers was measured using an electronic device comprising an impedance sensor as described above. The scalp moisture content for each consumer was determined as follows: One reading was taken from the forehead; and two readings were taken from the scalp. The measured moisture content values were then converted into a measured moisture content differential by the device. In addition, each consumer was assigned an Adherent Scalp Flaking Score ("ASFS"). The ASFS for each consumer was determined by having a qualified grader examine an octant of the consumer's scalp and then assigning a flake grade to that octant. Exemplary results are shown below Table II.

**TABLE II**

| Number of Consumers | Measured Signal Difference between m 1 and m2 (KHz) | ASFS Octant Grade | Scalp Condition |
|---|---|---|---|
| 61 | 395.164 | 8-10 | Severe Flaking |
| 235 | 308.766 | 4-6 | Moderate Flaking |
| 169 | 230.527 | 0-2 | No Flaking |

### Example 2. Method of Measuring Scalp Moisture Content of a Consumer

In one embodiment of the present invention, a consumer enters a retail location to shop for hair care products. While shopping, the consumer is greeted by a beauty counselor or hair care consultant, who asks consumer to provide some personal information about hair and scalp health conditions and/or preferences. The consumer is invited to sit at a computer terminal where she views and completes the questions of the questionnaire about, for example, benefits that are most important, whether or not she likes a cool and tingling feeling while washing hair and brand of hair care product currently being used. The consumer answers the questions by selecting one of multiple possible answers. The consumer then views information on the importance of scalp moisturization and signs of dandruff. Next, the consumer's scalp is tested using a moisture meter to obtain objective measurements on the moisture content of the scalp. A first reading is taken from the forehead of the consumer. A second reading is then taken from the scalp. The measurements from each reading are used with the personal information to recommend the appropriate hair care product for the consumer based on her individual needs and conditions. This information is then provided to the consumer by the beauty counselor or hair care consultant in the form of a product picture on the computer screen. As the beauty counselor or hair care consultant provides the details of the product recommendation to the consumer, the beauty counselor or hair care consultant may also provide the consumer with brochures or other educational information for her own individualized needs. The products recommended to the consumer in order to improve the health of the consumer's hair and scalp are available for her purchase in the retail location.

## Claims

1. A method for measuring scalp moisture content of a consumer, said method comprising the steps of:
a) collecting input information from said consumer regarding a plurality of characteristics associated with scalp condition of said consumer;
b) providing a moisture meter for measuring said scalp moisture content;
c) using said moisture meter to obtain at least one measured moisture content value from a position on scalp of said consumer; **characterized in that** the method further comprises the steps of:
d) obtaining a second measured moisture content value at a position off said scalp;
e) comparing said first measured moisture content value with said second measured moisture content value to obtain a measured moisture content differential;
f) correlating said measured moisture content differential to a scalp moisture value for said scalp;
g) using said input information and said scalp moisture value to identify at least one hair care product for said consumer; and
h) providing said consumer with information regarding the utilization of said at least one hair care product.

2. The method according to claim 1, wherein the step of recommending at least one hair care product includes an automated data analysis.

3. The method according to claim 2, wherein the automated data analysis is performed using software.

4. The method according to claim 1, wherein said input information is obtained from said consumer by questioning means.

5. The method according to claim 4, wherein said questioning means is an interview in a retail location.

6. The method according to claim 1, wherein said moisture meter comprises an impedance sensor.

7. The method according to claim 1, wherein said at least one hair care product is selected from the group consisting of shampoos, conditioners, oils, colorants, and styling compositions.

8. The method according to claim 1, wherein said information regarding said at least one hair care product is provided to said consumer through a written report.

9. The method according to claim 2, wherein said information regarding said at least one hair care product is provided to said consumer by means of graphical interface depicting the recommended at least one hair care product.

10. The method according to claim 9, wherein said graphical interface is outputted to an imaging device selected from the group consisting of a computer screen, television monitor, light radiating display and print materials.

## Patentansprüche

1. Verfahren zum Messen des Kopfhaut-Feuchtigkeitsgehaltes eines Verbrauchers, wobei das Verfahren die folgenden Schritte umfasst:
a) Sammeln von Eingabeinformationen des Verbrauchers in Bezug auf mehrere Charakteristiken, die mit dem Zustand der Kopfhaut des Verbrauchers in Zusammenhang stehen;
b) Bereitstellen eines Feuchtigkeitsmessers zum Messen des Kopfhaut-Feuchtigkeitsgehalts;
c) Verwenden des Feuchtigkeitsmessers, um von einer Position auf der Kopfhaut des Verbrauchers mindestens einen Feuchtigkeitsgehalt-Messwert zu erhalten; **dadurch gekennzeichnet, dass** das Verfahren ferner die folgenden Schritte umfasst:
d) Erhalten eines zweiten Feuchtigkeitsgehalt-Messwerts an einer nicht auf der Kopfhaut befindlichen Position;
e) Vergleichen des ersten Feuchtigkeitsgehalt-Messwerts mit dem zweiten Feuchtigkeitsgehalt-Messwert, um ein gemessenes Feuchtigkeitsgehaltsdifferential zu erhalten;
f) Korrelieren des gemessenen Feuchtigkeitsgehaltsdifferentials mit einem Kopfhautfeuchtigkeitswert für die Kopfhaut;
g) Verwenden der Eingabeinformationen und des Kopfhautfeuchtigkeitswertes zum Identifizieren mindestens eines Haarpflegeprodukts für den Verbraucher; und
h) Bereitstellen von Informationen gegenüber dem Verbraucher in Bezug auf die Verwendung des mindestens einen Haarpflegeprodukts.

2. Verfahren nach Anspruch 1, wobei der Schritt des Empfehlens mindestens eines Haarpflegeprodukts eine automatisierte Datenanalyse beinhaltet.

3. Verfahren nach Anspruch 2, wobei die automatisierte Datenanalyse unter Verwendung von Software ausgeführt wird.

4. Verfahren nach Anspruch 1, wobei die Eingabeinformationen von dem Verbraucher durch Befragungsmittel erhalten werden.

5. Verfahren nach Anspruch 4, wobei das Befragungsmittel ein Gespräch an einem Einzelhandelsstandort ist.

6. Verfahren nach Anspruch 1, wobei der Feuchtigkeitsmesser einen Impedanzsensor umfasst.

7. Verfahren nach Anspruch 1, wobei das mindestens eine Haarpflegeprodukt ausgewählt ist aus der Gruppe bestehend aus Shampoos, Haarspülungen, Ölen, Farbmitteln und Frisierzusammensetzungen..

8. Verfahren nach Anspruch 1, wobei die Informationen in Bezug auf das mindestens eine Haarpflegeprodukt dem Verbraucher durch einen schriftlichen Bericht bereitgestellt werden.

9. Verfahren nach Anspruch 2, wobei die Informationen in Bezug auf das mindestens eine Haarpflegeprodukt dem Verbraucher mittels einer grafischen Benutzeroberfläche bereitgestellt werden, die das empfohlene mindestens eine Haarpflegeprodukt darstellt.

10. Verfahren nach Anspruch 9, wobei die grafische Benutzeroberfläche an eine Abbildungsvorrichtung ausgegeben wird, die ausgewählt ist aus der Gruppe bestehend aus einem Computerbildschirm, einem Fernsehbildschirm, einer Licht abstrahlenden Anzeige und Druckmaterialien.

## Revendications

1. Procédé pour mesurer la teneur en humidité du cuir chevelu d'un consommateur, ledit procédé comprenant les étapes consistant à :
a) recueillir des informations d'entrée dudit consommateur concernant une pluralité de caractéristiques associées à l'état du cuir chevelu dudit consommateur ;
b) fournir un mesureur d'humidité pour mesurer ladite teneur en humidité du cuir chevelu ;
c) utiliser ledit mesureur d'humidité pour obtenir au moins une valeur de teneur en humidité mesurée d'une position sur le cuir chevelu dudit consommateur ; **caractérisé en ce que** le procédé comprend en outre les étapes consistant à :
d) obtenir une deuxième valeur de teneur en humidité mesurée à une position dudit cuir chevelu ;
e) comparer ladite première valeur de teneur en humidité mesurée à ladite deuxième valeur de teneur en humidité mesurée pour obtenir une différence de teneur en humidité mesurée ;
f) corréler ladite différence de teneur en humidité mesurée à une valeur d'humidité de cuir chevelu pour ledit cuir chevelu ;
g) utiliser lesdites informations d'entrée et ladite valeur d'humidité de cuir chevelu pour identifier au moins un produit de soin capillaire pour ledit consommateur ; et
h) fournir audit consommateur des informations concernant l'utilisation dudit au moins un produit de soin capillaire.

2. Procédé selon la revendication 1, dans lequel l'étape de recommandation d'au moins un produit de soin capillaire inclut une analyse automatisée des données.

3. Procédé selon la revendication 2, dans lequel l'analyse automatisée des données est effectuée en utilisant un logiciel.

4. Procédé selon la revendication 1, dans lequel lesdites informations d'entrée sont obtenues dudit consommateur par un moyen de questionnement.

5. Procédé selon la revendication 4, dans lequel ledit moyen de questionnement est un entretien dans un emplacement de vente au détail.

6. Procédé selon la revendication 1, dans lequel ledit mesureur d'humidité comprend un capteur d'impédance.

7. Procédé selon la revendication 1, dans lequel ledit au moins un produit de soin capillaire est choisi dans le groupe constitué de shampooings, conditionneurs, huiles, colorants, et compositions de coiffage.

8. Procédé selon la revendication 1, dans lequel lesdites informations concernant ledit au moins un produit de soin capillaire sont fournies audit consommateur par l'intermédiaire d'un rapport écrit.

9. Procédé selon la revendication 2, dans lequel lesdites informations concernant ledit au moins un produit de soin capillaire sont fournies audit consommateur au moyen d'une interface graphique représentant l'au moins un produit de soin capillaire recommandé.

10. Procédé selon la revendication 9, dans lequel ladite interface graphique est produite sur un dispositif d'imagerie choisi dans le groupe constitué d'un écran d'ordinateur, un moniteur de télévision, un affichage à rayonnement lumineux et des documents imprimés.
